# EUROPÄISCHE PATENTANMELDUNG

(11) **EP 1 920 793 A1**
(43) Veröffentlichungstag der Anmeldung: **14.05.2008**
(21) Anmeldenummer: 06123832.5
(22) Anmeldetag: 10.11.2006
(51) Int. Cl.: A61M 5/145

(54) **Optische Postitionserkennung des Stopfens einer Ampulle**

(71) Anmelder: F.HOFFMANN-LA ROCHE AG, 4070 Basel (CH); Roche Diagnostics GmbH, 68305 Mannheim (DE)
(72) Erfinder: Bosshard, David, 3251, Wengi (CH); Friedli, Kurt, 3421, Lyssach (CH); Stoller, Hanspeter, 3012, Bern (CH); Rufer, Thomas, 3072, Bern (CH); Ivan, Heutschi, 3506, Grosshöchstetten (CH); Haueter, Ulrich, 3506, Grosshöchstetten (CH)
(74) Vertreter: Schwabe - Sandmair - Marx

(57) **Zusammenfassung**

Die vorliegende Erfindung betrifft eine Vorrichtung zur Bestimmung des Füllstandes einer Substanz in einer Ampulle (7) mit mindestens einem an oder in der Nähe der Ampulle angeordneten lichtempfindlichen Sensor (1), mit welchem die Position eines Verdrängungskörpers (2) in der Ampulle, welche sich an oder in der Nähe des Sensors befindet, detektiert werden kann sowie ein Verfahren zur Bestimmung des Füllstandes einer Substanz in einer Ampulle mit mindestens einem lichtempfindlichen Sensor, wobei anhand des auf den lichtempfindlichen Sensor auftreffenden und von einem in der Ampulle enthaltenen Verdrängungskörper bezüglich seiner optischen Eigenschaften oder Intensität veränderten Lichtes die Position des Verdrängungskörpers, der Vorderseite, und/oder der Rückseite des Verdrängungskörpers ermittelt wird.

## Beschreibung

Die vorliegende Erfindung betrifft eine Vorrichtung und ein Verfahren zur Bestimmung des Füllstandes einer Ampulle, insbesondere zur Bestimmung der Füllmenge einer Substanz in einer Ampulle beziehungsweise in einem Reservoir, zum Beispiel im medizinischen Bereich in einem Medikamentenreservoir eines Infusionsgerätes, wobei der Anwendungsbereich hauptsächlich in der Überwachung und Regelung von Medikamentenpumpen des Spritzenpumpen-Typs, wie extrakorporalen Insulinpumpen, oder anderen Dosiersystemen, wie Medikamentenpens oder Dialysatampullen für die Mikrodialyse, liegt.

Bei der medizinischen Anwendung von Infusions- oder Injektionsgeräten ist die exakte Dosierung und Abgabe einer Substanz, welche sowohl fest als auch flüssig sein kann, wie zum Beispiel Insulin, Hormonpräparate oder ähnliches, wichtig. Hierzu wird häufig der Füllstand beziehungsweise die Füllmenge der Substanz in einer in ein Infusions- oder Injektionsgerät eingesetzten oder einsetzbaren Ampulle bestimmt. Zum einen sollte in einem solchen Gerät überwacht werden können, ob die eingestellte Substanzmenge abgegeben wurde oder überhaupt zur Abgabe bereit steht. Zum anderen sollte festgestellt werden können, wie der Füllstand der Ampulle beziehungsweise des Reservoirs ist.

Die US 6,362,591 B1 bezieht sich auf eine Vorrichtung und ein Verfahren zur automatischen Detektion einer Okklusion oder einer Fehlfunktion eines Antriebssystems in einem medizinischen Infusionssystem. Dabei wird der elektrische Strom zu einer Infusionspumpe gemessen und bei Überschreiten eines Schwellwertes ein Alarm ausgelöst, worauf anschließend der Motor der Pumpe zurückgefahren wird, um den Flüssigkeitsdruck im System abzubauen.

Die DE 198 40 992 A1 betrifft ein Verfahren zur Überwachung des Drucks eines bei einer Infusion oder Injektion dosiert zu verabreichenden Produktfluids, welches aus einem Behältnis durch Vorschieben eines Kolbens ausschüttbar ist Hierbei wird eine vom Kolben auf das Gehäuse ausgeübte Reaktionskraft gemessen und einer Steuerung für einen Antrieb des Kolbens zugeführt, welche den Antrieb des Kolbens unter der Berücksichtigung eines Vergleichsergebnisses steuert.

Aus der US 6,542,350 B1 ist ein Gerät zur Abgabe eines Medikaments bekannt, bei welchem nur das Volumen eines Behälters mittels eines Kondensators gemessen wird, wobei sich die Kapazität des Kondensators mit der Position eines Faltenbalges verändert.

Die DE 10 2004 040 441 A1 der Anmelderin offenbart eine Vorrichtung zur Bestimmung des Füllstandes einer Substanz in einer Ampulle mit mindestens zwei Elektroden, zwischen welchen die Substanz eingebracht werden kann; sowie ein Verfahren zur Bestimmung des Füllstandes einer Substanz in einer Ampulle mit mindestens zwei Elektroden, wobei die Bestimmung des Füllstandes durch eine Messung der Kapazität mindestens eines durch die mindestens zwei Elektroden gebildeten Kondensators durchgeführt wird, wobei die Dielektrizitätskonstante der zumindest zum Teil zwischen den mindestens zwei Elektroden liegenden Substanz die kapazitive Kopplung der mindestens zwei Elektroden beeinflusst.

Es ist eine Aufgabe der vorliegenden Erfindung, eine Vorrichtung und ein Verfahren zur einfachen und genauen Bestimmung des Füllstandes oder der Füllmenge einer Substanz in einer Ampulle beziehungsweise in einem Reservoir zu ermöglichen.

Diese Aufgabe wird durch die Gegenstände der unabhängigen Ansprüche gelöst Vorteilhafte Ausführungsformen ergeben sich aus den Unteransprüchen.

Die erfindungsgemäße Vorrichtung zur Bestimmung des Füllstandes einer Ampulle weist mindestens einen in der Nähe, an oder auch auf der Ampulle angeordneten lichtempfindlichen Sensor auf, mit welchem der Füllstand der Ampulle, insbesondere die Menge einer in einer Ampulle noch vorhandenen Substanz detektiert werden kann, zum Beispiel indem die Position eines Verdrängungskörpers, wie zum Beispiel eines Stopfens, in der Ampulle ermittelt wird. Dabei ist bevorzugt die Relativposition zwischen der Ampulle und dem lichtempfindlichen Sensor bekannt, das heißt, dass der lichtempfindliche Sensor zum Beispiel in einem definierten Lageverhältnis relativ zur Ampulle angeordnet ist, wenn diese definiert in eine Infusionsvorrichtung zum Beispiel bis zu einem Anschlag eingesetzt wird. Falls der lichtempfindliche Sensor direkt an dem Ampullenkörper angebracht ist, kann das relative Lageverhältnis bereits beim Herstellungsverfahren der Ampulle festgelegt werden. Der in der Ampulle vorhandene und verschiebbare Verdrängungskörper oder Stopfen ist vorzugsweise aus einem lichtundurchlässigen oder einem nur bestimmtes Licht durchlassenden Material und ist vorzugsweise nicht aus einem Material, welches Licht unverändert durchlässt, so dass anhand des sich durch die optischen Eigenschaften des Verdrängungskörpers veränderten auf den lichtempfindlichen Körper auftreffenden Lichtes festgestellt werden kann, wo sich der Verdrängungskörper und bevorzugt wo sich dessen substanzseitige Grenzfläche relativ zu dem lichtempfindlichen Sensor befindet. Ist weiterhin das Lageverhältnis zwischen dem lichtempfindlichen Sensor und der Ampulle, in welcher der Verdrängungskörper unter Abgabe einer Substanz verschoben wird, bekannt, so kann hieraus die relative Lage oder Position des Verdrängungskörpers oder Stopfens in der Ampulle ermittelt werden. Aus dieser Information kann bei bekannter Ampullengeometrie berechnet werden welche Menge einer Substanz noch in der Ampulle vorhanden ist oder welches Substanzvolumen abgegeben wird oder abgegeben worden ist.

Erfindungsgemäß kann somit in einer Ampulle oder allgemein in einem Reservoir der Füllstand präzise detektiert werden und beispielsweise eine in der Ampulle enthaltene verbleibende Medikamentenmenge gemessen werden, um zum Beispiel eine Dosiereinheit zu steuern. Hierdurch kann eine präzise Abgabe der Medikamentenmenge durch die exakt detektierte Stopfenposition realisiert werden.

Insbesondere ist es erfindungsgemäß möglich mit großer Präzision die Menge einer abzugebenden Substanz zu messen und auch die Abgabemenge präzise zu steuern, so dass insbesondere bei Anwendungsbereichen mit geringen Dosiermengen, wie zum Beispiel Kindern oder Typ II Diabetikern, die gewünschten geringen Dosismengen genau messbar abgegeben werden können.

Die direkte Messung der Stopfenposition ermöglicht darüber hinaus auch noch die genaue Kontrolle der in der Ampulle enthaltenen oder zum Beispiel aus der Ampulle bereits abgegebenen Substanz.

Vorzugsweise ist der mindestens eine lichtempfindliche Sensor in Längsrichtung oder längsseits der Ampulle vorgesehen, wenn diese zum Beispiel in eine Infusionsvorrichtung eingesetzt ist, und weist eine Länge auf, welche etwa oder genau der Ampullenlänge entspricht. Optional kann der lichtempfindliche Sensor auch so ausgebildet sein, dass er zum Beispiel von der Position der distalen Stopfenseite bei eingesetzter gefüllter Ampulle bis zur Position der distalen Stopfenseite bei einer teilweise oder auch vollständig entleerten Ampulle reicht.

Der mindestens eine lichtempfindliche Sensor kann ein elektronischer Sensor sein, welcher den äußeren oder inneren fotoelektrischen Effekt nutzt. Beispielsweise kann der lichtempfindliche Sensor als Fotozelle, Halbleiterdetektor, wie zum Beispiel Fotodiode, Fototransistor oder Fotowiderstand ausgebildet sein. Bevorzugt ist der mindestens eine lichtempfindliche Sensor als Serfsor-Array ausgestaltet und kann beispielsweise ein Zeilensensor (Line-Array) oder ein Flächensensor (Matrix-Array) sein, wie er beispielsweise aus Scannern oder Digitalkameras bekannt ist. Insbesondere ist der Sensor als PSD (Position Sensing Detector) oder CCD (Charge Coupled Device) ausgebildet.

Erfindungsgemäß kann der Sensor sowohl anhand von zum Beispiel Umgebungslicht detektieren, wo sich ein Verdrängungskörper in einer Ampulle befindet. Es ist jedoch auch möglich, dass eine Lichtquelle oder ein Beleuchtungselement an oder in der Nähe der Ampulle vorgesehen ist, welches Licht ausstrahlt, das zumindest zum Teil auf den mindestens einen lichtempfindlichen Sensor trifft, wobei zum Beispiel ein Teil des von der Lichtquelle ausgesandten Lichtes von dem Verdrängungskörper oder Stopfen absorbiert oder zum Beispiel nur zum Teil durchgelassen wird, um die Position des Verdrängungskörpers und insbesondere die Position der Stopfenvorderkante und/oder -hinterkante anhand des oder der Signale des mindestens einen lichtempfindlichen Sensors zu detektieren

Das Beleuchtungselement kann beispielsweise eine Leuchtdiode (LED) oder eine Leuchtdiodenanordnung sein, welche an oder zum Beispiel auch auf der Ampulle vorgesehen ist. Ebenso kann eine Glasfaservorrichtung entlang der Ampulle vorgesehen sein, welche beispielsweise auch in die Ampulle integriert sein kann und in welche zum Beispiel im eingesetzten Zustand der Ampulle an einer oder mehreren Seiten Licht eingespeist wird, welches zum Beispiel über eine in Längsrichtung der Ampulle aufgeraute Oberfläche so abgegeben wird, dass beispielsweise ein leuchtender Streifen entlang der Ampulle erzeugt werden kann. Vorzugsweise sind der mindestens eine lichtempfindliche Sensor und die Beleuchtungsvorrichtung so in einer Infusionsvorrichtung angeordnet, dass zumindest ein Teilstück der Ampulle oder des Verdrängungskörpers zwischen diesen liegt, wenn die Ampulle in eine Infusionsvorrichtung eingesetzt wird

Gemäß einer bevorzugten Ausführungsform der vorliegenden Erfindung ist die erfindungsgemäße Vorrichtung zur Bestimmung des Füllstandes einer Substanz mit einer Ampulle ausgestattet, in welcher die Substanz enthalten sein kann oder die Substanz eingebracht werden kann. Dabei kann die Ampulle in einem zum Beispiel als Einweg-Gerät ausgebildeten Infusionsgerät enthalten sein, also zum Beispiel fest mit diesem verbunden sein, oder kann in das als Mehrweg-Gerät ausgebildete Infusionsgerät eingebracht und wieder entfernt werden.

Bevorzugt weist die Ampulle ein Erkennungselement auf, welches Daten, wie zum Beispiel ampullenindividuelle Kalibrierdaten, enthalten beziehungsweise speichern kann. Dieses Erkennungselement kann besonders einfach als ein bestimmtes charakteristisches Oberflächenprofil auf der Ampulle ausgebildet sein, welches zum Beispiel auf Grund von Vertiefungen oder Erhebungen der Oberfläche der Ampulle Rückschlüsse auf die gespeicherten Daten zulässt. Die Kalibrierdaten können jedoch auch zum Beispiel in einem aus dem Stand der Technik bekannten Barcode gespeichert sein, aus welchem die Daten, wie bekannt, zum Beispiel mit dem mindestens einen lichtempfindlichen Sensor ausgelesen werden können. Auf der Ampulle kann auch ein Transponder als Erkennungselement vorgesehen sein, welcher die gespeicherten ampullenindividuellen Informationen bei Bedarf sendet. Bei den enthaltenen beziehungsweise gespeicherten Informationen kann es sich um jegliche Art von Daten handeln, die zur Kalibrierung der Ampulle beziehungsweise des Infusionsgerätes verwendet werden können, wie zum Beispiel die Größe, die Form, das Material oder die Aufnahmekapazität der verwendeten Ampulle, so dass zum Beispiel aus dem Weg, welchen die Stopfenvorderkante zurückgelegt hat, das Volumen der abgegebenen Substanz berechnet werden kann.

Gemäß einer weiteren bevorzugten Ausführungsform der Erfindung umfasst die Vorrichtung zur Bestimmung des Füllstandes einer Substanz in einer Ampulle ein Infusionsgerät, in welchem die Ampulle enthalten ist oder in welches die Ampulle eingebracht werden kann. Dieses Infusionsgerät kann eine Medikamentenpumpe des Spritzenpumpen-Typs, wie extrakorporale Insulinpumpen, oder ein anderes Dosiersystem sein, wie zum Beispiel Medikamentenpens oder Dialysatampullen für die Mikrodialyse.

Vorzugsweise weißt das Infusionsgerät oder die Infusionspumpe eine Dosiereinheit auf, mit welcher die Menge der aus einer Ampulle abgegebenen und zum Beispiel unter Druck stehenden oder durch einen Verdrängungskörper mit Druck beaufschlagten Substanz festgelegt und beispielsweise geregelt werden kann. Die Dosiereinheit kann in der einfachsten Form als Dosierventil oder auch als Dosiermechanik ausgebildet sein, womit zum Beispiel ein definiertes oder Referenzvolumen ein- oder mehrmals hintereinander abgegeben werden kann.

Wird der Verdrängungskörper oder Stopfen in der Ampulle zum Beispiel durch einen Antriebsmotor oder ein anderes Druckbeaufschlagungselement, wie zum Beispiel eine vorgespannte Feder, mit Druck beaufschlagt, so kann durch die Dosiereinheit unmittelbar festgelegt werden welche Menge der Substanz aus der Ampulle abgegeben wird, indem die Dosiereinheit vor der Abgabe der definierten Substanzmenge oder des Referenzvolumens geöffnet wird oder zum Beispiel den Weitertransport eines Referenzvolumens zu einer Ausgabeöffnung ermöglicht, wobei die Dosiereinheit nach der Abgabe des Referenzvolumens oder der gewünschten Substanzmenge eine weitere Abgabe verhindert und zum Beispiel ein Dosierventil wieder geschlossen wird. Somit kann die Substanzabgabe unterbrochen werden, wenn sich zum Beispiel die distale oder Stopfenvorderseite an einer Position befindet, welche durch das mindestens eine lichtempfindliche Element festgestellt wurde, an welcher die vorgegebenen Menge der Substanz aus der Ampulle abgegeben worden ist.

Zur Okklusionserkennung kann zum Beispiel die Position der distalen oder Stopfenvorderseite detektiert werden und die mittels des lichtempfindlichen Sensors detektierte Position der Stopfenvorderseite kann in Relation zu einer Stopfen-Soll-Position gesetzt werden. Die Stopfen-Soll-Position kann beispielsweise aus dem Ansteuersignal oder Ansteuerstrom eines den Stopfen antreibenden Motors berechnet werden und kann beispielsweise diejenige Soll-Position sein, welche der Stopfen einnehmen müsste, wenn im okklusionsfreien Zustand der Stopfen durch die Motorbewegung um einen definierten Weg vorgeschoben wurde und eine in der Ampulle enthaltene Substanz ungehindert abgegeben werden konnte. Liegt eine Okklusion oder Störung der Substanzabgabe zum Beispiel durch Verstopfen eines Katheters vor, so kann aus einer von der Stopfen-Soll-Position abweichenden mittels des mindestens einen lichtempfindlichen Sensors detektierten Stopfens-Ist-Position festgestellt werden, dass die gewünschte Substanzmenge oder das Substanzvolumen nicht oder nicht vollständig abgegeben wurde, so dass beispielsweise ein Okklusions-Warnsignal von der Inklusionsvorrichtung ausgegeben werden kann.

Alternativ oder ergänzend kann eine Okklusion beispielsweise auch durch die Detektion der distalen und proximalen Stopfenseite bzw. der Stopfenvorder- und Stopfenrückseite festgestellt werden, wenn beispielsweise ein kompressibles Medium, wie zum Beispiel ein elastisches Material oder Gummi, als Stopfen verwendet wird. Im Falle einer Okklusion wird ein zum Beispiel von einem Motor mit Druck beaufschlagter Stopfen zusammengedrückt, wenn die Druckbeaufschlagung des Stopfens nicht zu einer Abgabe der Substanz aus der Ampulle führt, so dass anhand des Vergleichs der Position der Stopfenvorderseite mit der Position der Stopfenrückseite ermittelt werden kann, dass eine Stopfen-Kompression vorliegt, so dass beispielsweise ein Okkulsians-Warnsignal ausgegeben werden kann

Gemäß einem weiteren Aspekt bezieht sich die Erfindung auf ein Verfahren zur Bestimmung des Füllstandes einer Substanz in einer Ampulle mit mindestens einem lichtempfindlichen Element, beispielsweise einem Licht-Sensor, wobei die Bestimmung des Füllstandes der Ampulle durch eine Messung des Lichtes, insbesondere der Lichtintensität, welche auf den Licht-Sensor einfällt, durchgeführt wird, wobei der in der Ampulle liegenden Verdrängungskörper oder Stopfen das auf den lichtempfindlichen Sensor auftreffende Licht oder die Lichtintensität beeinflusst

Gemäß einem weiteren Aspekt der Erfindung kann die Art einer in einer Ampulle enthaltenen Substanz bestimmt werden, wobei aus der optischen Eigenschaft, wie beispielsweise einem Transmissionswert der in der Ampulle enthaltenen Substanz, mittels beispielsweise eines Leuchtelements, das auf oder durch die Substanz hindurch leuchtet und mittels eines Sensors, der das von dem Leuchtelement ausgesandte und durch die Substanz optisch zum Beispiel beeinflusste, wie beispielsweise geschwächte oder ein bestimmtes Spektrum aufweisende Licht, aufnimmt, festgestellt wird, welche Substanz in der Ampulle enthalten ist.

Die Erfindung wird nachfolgend anhand eines Ausführungsbeispieles beschrieben werden. Es zeigt:

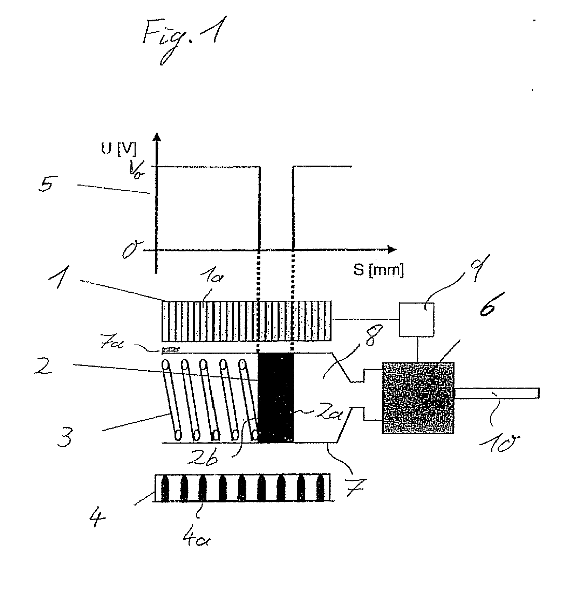

Figur 1 Eine Ausführungsform einer erfindungsgemäßen Stopfenpositions-Erkennung mit einem lichtempfindlichen Sensor.

Figur 1 zeigt eine Ampulle 7, in welcher ein Stopfen 2 angeordnet ist, der durch eine Feder 3, welche sich beispielsweise an einer Infusionsvorrichtung abstützt, in welcher die Ampulle 7 eingesetzt ist, mit Druck beaufschlagt wird. An der Seite der Ampulle 7 sind in Längsrichtung der Ampulle 7 ein Beleuchtungselement 4 bestehend aus mehreren nebeneinander angeordneten Leuchtdioden 4a und auf der gegenüberliegenden Seite der Ampulle 7 ein optischer Sensor 1 aus mehreren nebeneinander liegenden einzelnen unabhängig voneinander optische Signale aufnehmenden Licht-Detektions-Elementen 1a mit einer jeweiligen Breite von 1µm vorgesehen.

Auf der Seite der Ausgabeöffnung der Ampulle 7 ist eine Dosiereinheit 6 vorgesehen, in weicher sich ein Dosierventil befindet, welches sich von einer Steuerung 9 in Abhängigkeit von zum Beispiel einem Bedienungssignal oder einer mittels des Sensor 1 detektierten Stopfenposition öffnen oder schließen lässt. An der Dosiereinheit 6 ist ein Katheter 10 vorgesehen, durch welchen die in der Ampulle 7 enthaltene Substanz an einen Patienten abgegeben wird.

In der in Figur 1 gezeigten Position des Stopfens 2 wird von dem Sensor 1 das Signal U [V] abgegeben, welches in Abhängigkeit von der Position des Stopfens 2 in der Ampulle 7 in dem durch die Beleuchtung 4 beleuchteten Bereich des Sensors 1 den Signalwert VO hat und in dem von dem Stopfen 2 vor der Beleuchtung abgeschirmten Bereich den Wert 0 annimmt, so dass der Schatten des Stopfens 2 durch den Sensor 1 detektiert werden kann.

Die distale Stopfenseite oder vordere Stopfenkante 2a des Stopfens 2 seitens des Medikamentes ist somit an der Position, an welcher die Flanke des Signales U[V] wieder ansteigt und wird zum Beispiel von dem auf den Sensor 1 auftreffenden von der Beleuchtungsvorrichtung 4 stammenden Photonenstrom detektiert, welcher in das gezeigte elektrische Signal U[V] umgewandelt wird.

Durch die Druckbeaufschlagung des Stopfens 2 mit der Feder 3 kann beispielsweise ein definierter Systemdruck erzeugt werden. Löst ein Benutzer zum Beispiel durch Betätigung eines an einer Infusionsvorrichtung vorgesehenen Betätigungselementes einen Infusionsvorgang aus, um zum Beispiel einen Bolus abzugeben, oder soll im Wege der Basal-Abgabe der Stopfen 2 um eine definierte Weglänge nach vorne bewegt werden, so kann das in der Dosiereinheit 6 enthaltene Dosierventil so lange geöffnet werden, bis die durch den Sensor 1 detektierte Position der Stopfenvorderkante 2a an der gewünschten Position ist, an welcher die gewünschte Substanzmenge abgegeben wurde. Wird von dem Sensor 1 die Stopfenvorderseite 2a an der gewünschten Position oder an einer zum Beispiel aufgrund eines Fehlers noch weiter eingeschobenen Position detektiert, so kann die Dosiereinheit 6 die weitere Abgabe einer Substanz verhindern, indem beispielsweise das in der Dosiereinheit 6 enthaltene Dosierventil wieder geschlossen wird.

Im Vergleich zu Pumpen mit einer Motorsteuerung kann somit das Gesamtsystem miniaturisiert werden, da es zur Steuerung der Substanzabgabe erfindungsgemäß ausreicht die Stopfenposition zu detektieren und zum Beispiel nicht mittels eines Motors eine definierte Kraft aufgebracht oder eine definierte Vorschubbewegung des Stopfens realisiert werden muss Somit lassen sich beispielsweise Infusionspumpen ohne Motor und Getriebe realisieren.

Es wird angemerkt, dass das unter Bezugnahme auf Figur 1 geschilderte Prinzip auch bei herkömmlichen Fördersystemen mit Schrittmotor und Teleskopstange angewendet werden kann.

Soll außer der mit der Erfindung möglichen Füllstandkontrolle der Ampulle 7 auch noch eine Okklusionserkennung realisiert werden, kann beispielsweise die Sollposition eines Antriebsflansches, welcher den Stopfen 2 vorantreibt, detektiert werden und mit der Position der Vorderkante 2a des Stopfens 2 in Korrelation gebracht werden, so dass erkannt werden kann, wenn der Stopfen 2 komprimiert wurde. Anschließend kann ein Warnsignal ausgegeben werden und beispielsweise die Medikamentenförderung angepasst werden. Das heißt es kann beispielsweise anhand der Position der Stopfenvorderseite 2a festgestellt werden, dass eine zu geringe Substanzmenge abgegeben wurde, wobei die Differenz zur gewünschten abzugebenden Substanzmenge auch quantifiziert werden kann. Nach der Behebung der Okklusion kann beispielsweise diese fehlende Substanzmenge noch zusätzlich abgegeben werden, falls dies gewünscht und zum Beispiel von einem Benutzer veranlasst wird.

## Patentansprüche

1. Vorrichtung zur Bestimmung des Füllstandes einer Substanz (8) in einer Ampulle (7) mit mindestens einem an oder in der Nähe der Ampulle (7) angeordneten lichtempfindlichen Sensor (1), mit welchem die Position eines Verdrängungskörpers (2) in der Ampulle (7), welche sich an oder in der Nähe des Sensors (1) befindet, detektiert werden kann.

2. Vorrichtung nach Anspruch 1 mit mindestens einem Beleuchtungselement (4), welches an einer Ampulle (7) oder in der Nähe einer Ampulle (7) angeordnet ist.

3. Vorrichtung nach Anspruch 2, wobei der mindestens eine Sensor (1) und das mindestens eine Beleuchtungselement (4) relativ zueinander so angeordnet sind, dass zwischen dem mindestens einen Sensor (1) und dem mindestens einen Beleuchtungselement (4) eine Ampulle (7) eingesetzt ist oder eingesetzt werden kann.

4. Vorrichtung nach einem der Ansprüche 2 oder 3, wobei der mindestens eine Sensor (1) und/oder das mindestens eine Beleuchtungselement (4) länglich ausgebildet sind und insbesondere die Länge einer Ampulle (7) aufweisen.

5. Vorrichtung nach einem der vorhergehenden Ansprüche, wobei das mindestens eine lichtempfindliche Element (1) mehrere benachbarte lichtempfindliche Sensoren aufweist oder ein Charge Coupled Device (CCD) ist.

6. Vorrichtung nach einem der vorhergehenden Ansprüche, wobei das mindestens eine Beleuchtungselement (4) eine Leuchtdiode oder Leuchtdiodenanordnung oder ein Glasfaserelement ist.

7. Vorrichtung nach einem der vorhergehenden Ansprüche mit einer Ampulle (7).

8. Vorrichtung nach dem vorhergehenden Anspruch, wobei ein Verdrängungskörper oder Stopfen (2) der Ampulle (7) aus einem lichtundurchlässigen oder hindurchtretendes Licht beeinflussenden Material besteht.

9. Vorrichtung nach einem der zwei vorhergehenden Ansprüche, wobei die Ampulle (7) ein Erkennungselement (7a), insbesondere einen Transponder, einen Barcode oder ein charakteristisches Oberflächenprofil aufweist, weiches ampullenindividuelle Daten und insbesondere Kalibrierdaten speichern kann.

10. Infusionsvorrichtung mit einer Vorrichtung zur Bestimmung des FÜllstandes einer Substanz (8) in einer Ampulle (7) nach einem der vorhergehenden Ansprüche.

11. Infusionsvorrichtung nach dem vorhergehenden Anspruch, wobei der mindestens eine lichtempfindliche Sensor (1) und das mindestens eine Beleuchtungselement (4) auf entgegengesetzten Seiten und längsseits einer Ampullen-Einsetz-Öffnung vorgesehen sind.

12. Infusionsvorrichtung nach einem der zwei vorhergehenden Ansprüche, wobei die Infusionsvorrichtung eine Leseeinheit (1) und/oder eine Auswerteeinheit zum Auslesen und/oder Auswerten der Kalibrierdaten des mindestens einen Erkennungselementes (7a) umfasst.

13. Infusionsvorrichtung nach einem der drei vorhergehenden Ansprüche mit einer Dosiereinheit (6), insbesondere einem Dosierventil oder einer Dosiermechanik, welche im Bereich einer Abgabeöffnung einer vorhandenen oder einzusetzenden Ampulle (7) vorgesehen ist und welche mit der Ampulle (7) gekoppelt werden kann.

14. Infusionsvorrichtung nach einem der vier vorhergehenden Ansprüche mit einem Steuerelement (9) zum Ansteuern der Dosiereinheit (6), wobei die Steuereinheit (9) mit dem lichtempfindlichen Sensor (1) verbunden ist und an die Dosiereinheit (6) ein Stop-Signal ausgeben kann, wenn von dem mindesten einen lichtempfindlichen Sensor (1) detektiert wird, dass die Vorderkante (2a) des Verdrängungskörpers oder Stopfens (2) an einer vorgegebenen Position ist.

15. Infusionsvorrichtung nach einem der fünf vorhergehenden Ansprüche mit einem Motor und/oder einer Feder (6) zum Antreiben oder zur Druckbeaufschlagung des Verdrängungskörpers oder Stopfens (2).

16. Infusionsvorrichtung nach einem der sechs vorhergehenden Ansprüche mit einer Okklusionserkennungseinheit, welche mit dem Sensor (1) gekoppelt ist und anhand des Vergleichs der von dem lichtempfindlichen Sensor (1) detektierten Positionen der Vorderseite (2a) und der Rückseite (2b) des Stopfens (2) detektieren kann, ob der Stopfen (2) komprimiert wurde und eine Okklusion vorliegt

17. Infusionsvorrichtung nach einem der sieben vorhergehenden Ansprüche, wobei eine Okklusionserkennungseinheit mit dem mindestens einen lichtempfindlichen Sensor (1) und einem den Stopfen (2) antreibenden Motor gekoppelt ist, wobei durch einen Vergleich der aus dem Motor-Ansteuer-Strom ermittelten Stopfen-Soll-Position mit einer mittels des lichtempfindlichen Sensor (1) ermittelten Stopfen-Ist-Position ermittelt wird, ob eine Okklusion vorliegt.

18. Verfahren zur Bestimmung des Füllstandes einer Substanz in einer Ampulle (7) mit mindestens einem lichtempfindlichen Sensor (1), wobei anhand des auf den lichtempfindlichen Sensor (1) auftreffenden und von einem in der Ampulle (7) enthaltenen Verdrängungskörper (2) bezüglich seiner optischen Eigenschaften oder Intensität veränderten Lichtes die Position des Verdrängungskörpers (2), der Vorderseite (2a), und/oder der Rückseite (2b) des Verdrängungskörpers (2) ermittelt wird.

19. Verfahren zur Okklusions-Detektion, wobei aus der mittels eines lichtempfindlichen Sensors (1) detektierten Position der Vorderseite (2a) und der Rückseite (2b) eines Verdrängungskörpers (2) einer Ampulle (7) ermittelt wird, ob der Verdrängungskörper (2) komprimiert wurde und eine Okklusion vorliegt.

20. Verfahren zur Detektion einer Okklusion bei einer durch einen Motor angetriebenen Infusionsvorrichtung, wobei durch einen Vergleich der aus einem Motor-Ansteuer-Strom ermittelten Soll-Position eines Stopfens (2) mit einer mittels eines lichtempfindlichen Sensors (1) ermittelten Ist-Position des Stopfens (2) ermittelt wird, ob eine Okklusion vorliegt.

21. Verfahren zur Bestimmung der Art einer Substanz, wobei aus einem gemessenen Transmissionswert einer zwischen mindestens einem Beleuchtungselement (4) und mindestens einem Sensor (1) enthaltenen Substanz ermittelt wird, welche Art der Substanz vorliegt
